# EUROPÄISCHE PATENTSCHRIFT

(11) **EP 1 200 051 B1**
(45) Veröffentlichungstag und Bekanntmachung des Hinweises auf die Patenterteilung: **01.02.2006**
(21) Anmeldenummer: 00963997.2
(22) Anmeldetag: 02.08.2000
(51) Int. Cl.: A61K 8/41, A61K 8/49, A61Q 5/10

(54) **MITTEL ZUM FÄRBEN VON KERATINHALTIGEN FASERN**
AGENT FOR DYING KERATIN FIBERS
AGENT POUR TEINDRE DES FIBRES KERATINIQUES

(30) Priorität: 05.08.1999 DE 19936911
(43) Veröffentlichungstag der Anmeldung: 02.05.2002
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE); HÖFFKES, Horst, 40595 Düsseldorf (DE)
(86) Internationale Anmeldenummer: PCT/EP2000/007163
(87) Internationale Veröffentlichungsnummer: WO 2001/013866

(56) Entgegenhaltungen:
- EP-A- 0 502 783
- EP-A- 0 820 759
- WO-A-00/33799
- WO-A-00/38368
- WO-A-00/38369
- DE-A- 19 820 894
- FR-A- 2 787 707
- FR-A- 2 787 708
- US-A- 5 034 014

## Beschreibung

Die Erfindung betrifft ein Mittel zum Färben von keratinhaltigen Fasern, insbesondere menschlichen Haaren, das eine Kombination aus aromatischen Aldehyden bzw. Ketonen und CH-aktiven Verbindungen enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren. Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin. p-Toluylendiamin, 2,4,5,6-Tetraaminopyrimidin, p-Aminophenol. N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

WO 00/38639, veröffentlicht am 06.07.2000, offenbart Färbemittel enthaltend 4-Dimethylaminobenzaldehyd in Kombination mit den CH-aktiven Verbindungen 3-Ethyl-methylbenzothiazolium-iodid, 3-Ethylmethylbenzoxazolium-iodid, 1,2-Dimethylchinolinium-iodid, 5-Chlor-3-ethyl-2-methylbenzothiazolium-iodid, 2-Methyl-1-(3-sulfopropyl)-naphtho[1,2-d]-thiazolium-betain und 5-Methoxy-2-methyl-3-(3-sulfopropyl)-benzothiazolium-betain. WO 00/38638, veröffentlicht am 06.07.2000, offenbart ein Färbemittel mit einer Kombination aus 4-Dimethylaminobenzaldehyd und 3-Amino-1-phenyl-pyrazolin-5-on.

Die Verwendung der unten näher beschriebenen Kombination aus aromatischen Aldehyden bzw. Ketonen und CH-aktiven Verbindungen zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die Kombination aus den in der Formel I dargestellten aromatischen Aldehyden beziehungsweise Ketonen und CH-aktiven Verbindungen der Formeln 11 und/oder III sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend eine Kombination aus aromatischen Aldehyden bzw. Ketonen mit der Formel I, 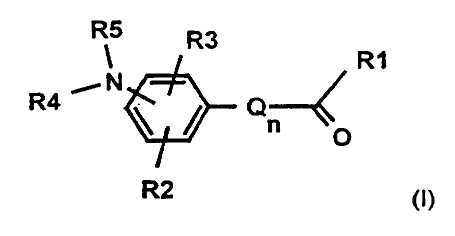 in der R¹ ein Wasserstoffatom, eine C₁₋₄-Alkyl- oder eine Arylgruppe ist,
R², R³ unabhängig voneinander ein Wasserstoffatom, eine C₁₋₄-Alkylgruppe, ein Halogenatom, eine Hydroxy-, C₁₋₄-Alkoxy-, Nitrogruppe oder zusammen einen ankondensierten aromatischen Ring bedeuten,
R⁴ und R⁵ ein Wasserstoffatom, eine C₁₋₄-Alkyl-, C₂₋₄-Hydroxyalkyl-, C₁₋₄-Alkenyl- oder Arylgruppe bedeuten oder gemeinsam mit dem Stickstoffatom einen fünf bis siebengliedrigen heterocylischen Ring bilden, oder einer von ihnen mit einem der Reste R² oder R³ einen gesättigten oder ungesättigten 5-, 6- oder 7-Ring bildet,
Q eine direkte Bindung oder eine Vinylen- oder eine Vinylidengruppe, die zusammen mit einem der beiden Reste R⁴ oder R⁵ einen Indolring oder bei Ausbildung einer exocyclischen Doppelbindung einen Indolinring bilden kann, und
n 1 oder 2 ist,
und CH-aktiven Verbindungen mit den Formeln II und/oder 111, 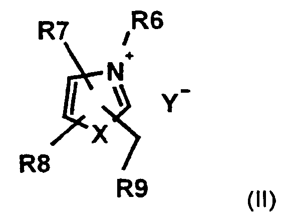
in der R⁶ eine C₁₋₁₀-Alkyl-, C₂₋₁₀-Alkenyl-, C₂₋₄-Hydroxyalkyl-, C₂₋₄-Carboxyalkyl-, C₂₋₄₋Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe >N-R¹², oder -CH=CH-, in denen
R¹² für eine C₁₋₄-Alkyl-, C₂₋₄-Carboxalkyl-, C₂₋₄-Sulfoalkyl-, C₂₋₄-Sulfoxyalkyl-, C₂₋₄₋Hydroxyalkyl- oder Aralkylgruppe, steht, ist und
Y⁻ für ein Anion steht, das ausgewählt ist aus Halogenid, C₁₋₄-Alkylsulfat, C₁₋₄₋Alkansulfonat, Arensulfonat, C₁₋₄-Perfluoralkansulfat, Tetrafluorborat, Perhalogenat, Sulfat, Hydrogensulfat oder Carboxylat, 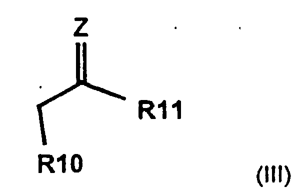 in der R¹⁰ für eine C₁₋₄-Acylgruppe, Aroyl-, C₁₋₄-Alkylsulfonyl-, C₁₋₄-Alkylsulfinyl-, C₁₋₄₋Alkylamino-, Di-C₁₋₄-Alkylamino-, Vinylcarbonyl-, Methinimino-, Nitril-, Ester- oder Carbonsäureamidgruppe, die ggf. durch C₁₋₄-Alkyl-, C₂₋₄-Hydroxyalkyl oder Arylgruppen substituiert sein kann, steht und
R¹¹ für eine C₁₋₄-Acyl-, C₁₋₄-Alkoxy-, C₁₋₄-Alkylamino-, C₁₋₄-Acylamino- oder Di-C₁₋₄₋alkylaminogruppe steht,
wobei die Reste R¹⁰ und R¹¹ gemeinsam mit dem Restmolekül einen 5-, 6- oder 7- gliedrigen Gyclus aus der Reihe der 1,3-Thiazolidin-2,5-dione, 1,3-Thiazolidin-2-thion-5-one, Perhydropyrimidin-2,4,6-trione, Perhydropyrimidin-2-thion-4,6-dione, Cyclopentan-1,3-dione, Cyclohexan-1,3-dione, Indoline, Indan-1,3-dione, Indan-1-one, 2-Pyrazolin-5-one, 1,2-Dihydro-6-hydroxy-2-hydroxypyridine oder deren Enolester bilden können, und Z für Sauerstoff, Schwefel oder die Dicyanmethylengruppe steht
und/oder Reaktionsprodukte aus diesen Verbindungen mit den Formeln I und II und/oder III, ausgenommen Mittel, enthaltend 4-Dimethylaminobenzaldehyd als Verbindung gemäß Formel (I) in Kombination mit einer der CH-aktiven Verbindungen, ausgewählt aus der Gruppe, die gebildet wird aus 3-Ethyl-methylbenzothiazolium iodid, 3-Ethylmethylbenzoxazolium iodid, 1,2-Dimethylchinolinium iodid, 5-Chlor-3-ethyl-2-methylbenzothiazolium iodid, 2-Methyl-1-(3-sulfopropyl)-naphtho[1,2-d]thiazolium betain, 5-Methoxy-2-methyl-3-(3-sulfopropyl)benzothiazolium betain und 3-Amino-1-phenyl-pyrazolin-5-on.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel I sind z. B. 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)benzaldehyd, 4-Diphenylamino-benzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-aldehyd sowie deren beliebigen Gemische.

Als Beispiele für bevorzugt eingesetzte Verbindungen mit der Formel II können genannt werden: 1,4-Dimethylchinolinium-, 1,2-Dimethylchinolinium-, 1,4-Dimethylpyridinium-, 1,2-Dimethylpyridinium-, 2-Methyl-1-ethylchinolinium-, 2,3-Di-methylisochinolinium-, 2,3-Dimethylbenzothiazolium-, 2,3-Dimethyl-6-nitrobenzothiazolium-, 3-Benzyl-2-benzothiazolium-, 2-Methyl-3-propylbenzothiazolium-, 2,4-Dimethyl-3-ethylthiazolium-, 3-(2-Carboxyethyl)-2,5-dimethylbenzothiazolium-, 1,2,3-Trimethylbenzimidazolium-, 5,6-Dichlor-1,3-diethyl-2-methylbenzimidazolium-, 3-Ethyl-2-methylbenzothiazolium-, 3-Ethyl-2-methylnaphtho[1,2-d]thiazolium-, 5-Chlor-3-ethyl-2-methylbenzothiazolium-, 3-Ethyl-2-methylbenzoxazolium-Salze, die z. B. als Chloride, Bromide, lodide, Methansulfonate, Benzolsulfonate, p-Toluolsulfonate, Trifluormethansulfonate, Methylsulfate, Tetrafluorborate vorliegen können, sowie 2-Methyl-3-(3-sulfopropyl)-benzothiazolium-hydroxid, inneres Salz, 5-Methoxy-2-methyl-3-(3-sulfopropyl)-benzothiazolium-hydroxid, inneres Salz und beliebige Gemische der voranstehenden.

Beispiele für Verbindungen mit der Formel III sind Rhodanin, Rhodanin-3-essigsäure, Barbitursäure, Thiobarbitursäure, 1,3-Dimethyl-, 1,3-Diethylthiobarbitursäure, Oxindol, 3-Indoxylacetat, 1-Methyl-3-phenylpyrazolinon, Indan-1,3-dion, Cyclopentan-1,3-dion, 1,2-Dihydro-1-ethyl-6-hydroxy-4-methyl-2-oxo-3-pyridincarbonitril und beliebige Gemische der voranstehenden.

Beispiele für Reaktionsprodukte der Verbindungen mit den Formel I und II und/oder III sind Quinaldine red (2-[4-(Dimethylamino)-styryl}-1-ethylchinolinium-iodid), 2-[4-(Dimethylamino)-styryl]-1-methylchinolinium-iodid, 2-[4-(Dimethylamino)-styryl]-1-ethylpyridinium-iodid, 2-[4-(Dimethylamino)-styryl]-1-methylpyridinium-iodid, 4-[4-Dimethylaminostyryl]-1-methylpyridinium-tosylat, 4-[2-(2,3,6,7-Tetrahydro-1H,5H-benzo[ij]chinolizin-9-yl)-ethenyl]-1-methylpyridinium-iodid, trans-4-[4-(Dibutylamino)-styryl]-1-methylpyridiniumioidid und 2,6-Bis-[4-(dimethylamino)-styryl]-1-methylpyridmium-iodid, 2-[4-(Dimethylamino)-styryl]-3-methylbenzoxazolium-perchlorat, 2-[4-(Dimethylamino)-styryl]-3-methylbenzoxazolium-iodid, 2-[4-(Dimethylamino)-styryl-1-ethylnaphtho[1.2-d]thiazolium-iodid.

Diese Substanzen sind zum großen Teil literaturbekannt oder im Handel erhältlich oder nach bekannten Syntheseverfahren herstellbar.

Die voranstehend genannten Verbindungen mit der Formel 1, Formel II bzw. Formel III werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können als direktziehende Färbemittel oder ih Gegenwart von üblichen Oxidationsfarbstoffvorprodukten eingesetzt werden.

Färbemittel, die als färbende Komponente die erfindungsgemäße Kombination allein enthalten, werden bevorzugt für Färbungen im Gelb-, Orange-, Rot- und Violettbereicheingesetzt.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Kombinationen aus den Verbindungen mit den Formeln I, II und/oder III gemeinsam zum Einsatz kommen; ebenso können auch mehrere verschiedene Farbverstärker gemeinsam verwendet werden.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot. Henna neutral, Henna schwarz, Kamillenblüte, Sandelholz, schwarzen Tee. Faulbaumrinde, Salbei, Blauholz, Krappwurzel, Catechu, Sedre und Alkannawurzel enthalten.

Es ist nicht erforderlich, daß die erfindungsgemäß enthaltenen Verbindungen mit der Formeln I und II oder die fakultativ enthaltenen Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die

Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 34.4,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂₋Carbonsäuren, wie Ölsäure, Stearinsäure, isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmittein verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homo- logenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrytamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, CelluloseDerivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure.
- haarkonditionierende Verbindungen wie Phospholipide, beispielsweise Sojalecithin, Ei-Lecitin und Kephaline, sowie Silikonöle,
- Proteinhydrolysate, insbesondere Elastin-. Kollagen-, Keratin-, Milcheiweiß-, Sojaprotein- und Weizenproteinhydrolysate deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 12, vorzugsweise zwischen 4 und 10.
Ein weiterer Gegenstand der vorliegenden Erfindung betrifft die Verwendung eines Färbemittels gemäß Ansprunch 1 als eine färbende kimponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel gemäß Anspruch 1 auf die keratinhaltigen Fasern aufgebracht, einige Zeit, üblicherweise ca. 30 Minuten, auf der Faser belassen und anschließend wieder ausgespült oder mit einem Shampoo ausgewaschen wird.
Die aromatischen Aldehyde bzw. Ketone der Formel I und die CH-aktiven Verbindungen mit den Formeln II und/oder III können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder

Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die aromatischen Aldehyde beziehungsweise Ketone der Formel I und die CH-aktiven Verbindungen mit den Formeln II und/oder III können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines aromatischen Aldehyds oder Ketons mit der Formel 1, 5 mmol einer CH-aktiven Verbindung mit der Formel II bzw. III in 25 ml Wasser bei 60°C hergestellt. Die Aufschlämmungen wurden nach Abkühlen auf 30°C miteinander vermischt, mit 5 mmol Natriumacetat, ggf. 5 mmol Piperidin und einem Tropfen einer 20-%igen Fettalkylethersuffat-Lösung versetzt und mit verdünnter NaOH oder Salzsäure der pH-Wert entsprechend eingestellt.

Wenn Reaktionsprodukte aus den Verbindungen der Formel I und Verbindungen der Formeln II bzw. 111 eingesetzt wurden, wurden 5 mMol dieses Reaktionsproduktes in 50 ml Wasser aufgeschlämmt oder gelöst.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in den nachfolgenden Tabellen wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **aromatischer Aldehyd bzw. Keton** | **Verbindung mit der Formel II oder III** | **Farbverstärker** | **pH** | **Farbe** | **Intensität** |
|---|---|---|---|---|---|
| 4-Dimethylaminobenzaldehyd | 1-Ethyl-2-methylchinolinium-iodid | Piperidin | 9,0 | violettrot | ++(+) |
| 4-Dimethylaminobenzaldehyd | 1,4-Dimethylchinoliniummethansulfonat | Piperidin | 9,0 | rotviolett | +(+) |
| 4-Dimethyl-aminobenzaldehyd | 3-Ethyl-2-methylbenzothiazolium-p-toluolsulfonat | Piperidin | 7,60 | leuchtend rosarot | +++ |
| 4-Dimethylaminobenzaldehyd | Rhodanin | Piperidin | 7,60 | leuchtend orange | ++(+) |
| 4-Dimethylaminobenzaldehyd | Oxindol | Piperidin | 9,00 | goldgelb | ++(+) |
| 4-Dimethylaminobenzaldehyd | 1,3-Diethylthiobarbitur-saeure | | 9,00 | orange | ++ |
| 4-Dimethylaminobenzaldehyd | 1-Ethyl-2-methylnaphtho[1,2-d]-thiazolium-p-toluolsulfonat | - | 6,00 | karminrot | ++(+) |
| 4-Dimethylamino-1-naphthaldehyd | 1-Ethyl-2-methylnaphtho[1,2-d]-thiazolium-p-toluolsulfonat | -- | 6,00 | dunkelviolett | ++(+) |
| 4-Dimethylamino-1-naphthaldehyd | 3-Ethyl-2-methylbenzothiazolium-p-toluolsulfonat | Piperidin | 7,73 | rotviolett | ++(+) |
| 4-Dimethylamino-1-naphthaldehyd | 1-Ethyl-2-methylchinolinium-iodid | Piperidin | 9,00 | violett-braun | +(+) |
| 4-Dimethylaminozimtaldehyd | 1-Ethyl-2-methylchinolinium-iodid | Piperidin | 9.00 | dunkelorangebraun | ++(+) |
| 4-Dimethylaminozimtaldehyd | 3-Ethyl-2-methylbenzothiazolium-p-toluolsulfonat | Piperidin | 7,33 | braun | ++ |
| 4-Dimethyl-aminozimtaldehyd | Rhodanin | -- | 6,00 | rotorange | ++ |
| 4-Dimethyl-aminozimtaldehyd | Rhodaninessigsäure | -- | 6,00 | orangerot | ++(+) |
| 4-Dimethyl-aminozimtaldehyd | 1,3-Diethylthiobarbitursaeure | -- | 9,00 | braunviolett | ++ |
| 4-Dimethylaminozimtaldehyd | 1,4-Dimethylchinoliniummethansulfonat | Piperidin | 9,00 | dunkelgelbbraun | ++ |
| 1-Methylindol-3-aldehyd | 1-Ethyl-2-methylnaphtho[1,2-d]-thiazolium-p-toluolsulfonat | -- | 6,00 | rotorange | ++(+) |
| indol-3-aldehyd | 1-Ethyl2-methylnaph-tho[1,2-d]-thiazolium-p-toluolsulfonat | -- | 6,00 | gelborange | + |
| Indol-3-aldehyd | 3-Ethyl-2-methylbenzolthizolium-iodid | -- | 6,00 | goldgelb | + |

**Tabelle 2 Ausfärbungen mit Reaktionsprodukten der Verbindungen mit der Formel I und II**

| **Reaktionsprodukt** | **Farbe** | **Intensität** | **pH-Wert** |
|---|---|---|---|
| 2-(4-Dimethylaminostyryl)-1-ethylpyridiniumiodid | leuchtend orange | +++ | 6,00 |
| 2-(4-Dimethylaminostyryl)-1-methylchinolinium-iodid | violettrot | ++(+) | 6,00 |
| 4-[(1-methyl-4(1H)-pyridinyliden)-ethyliden-2,5-cyclohexadien-1-on | orangegelb | +(+) | 6,00 |
| 3-(4-Dimethylaminobenzyliden)-2-indolinon | goldgelb | ++ | 6,00 |
| 5-(4-Dimethylaminobenzyliden)-5-thioxo-2-pyrrolidinon | leuchtend orange | ++ | 6,00 |
| 2-(4-Dimethylaminostyryl)-3-ethylbenzothiazolium-iodid | leuchtend orange | ++ | 6,00 |

## Patentansprüche

1. Mittel zum Färben von keratinhaltigen Fasern, insbesondere menschlichen Haaren, enthaltend als färbende Komponente eine Kombination aus aromatischen Aldehyden bzw. Ketonen mit der Formel I, 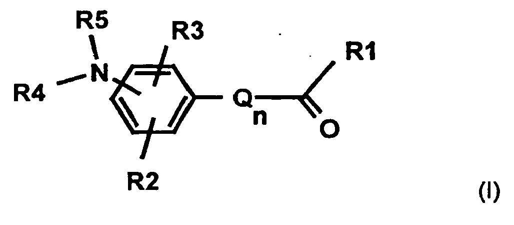
in der R¹ ein Wasserstoffatom, eine C₁₋₄-Alkyl- oder eine Arylgruppe ist,
R², R³ unabhängig voneinander ein Wasserstoffatom, eine C₁₋₄-Alkylgruppe, ein Halogenatom, eine Hydroxy-, C₁₋₄-Alkoxy-, Nitrogruppe oder zusammen einen ankondensierten aromatischen Ring bedeuten,
R⁴ und R⁵ ein Wasserstoffatom, eine C₁₋₄-Alkyl-, C₂₋₄-Hydroxyalkyl-, C₁₋₄-Alkenyl- oder Arylgruppe bedeuten oder gemeinsam mit dem Stickstoffatom einen fünf bis siebengliedrigen heterocylischen Ring bilden, oder einer von ihnen mit einem der Reste R² oder R³ einen gesättigten oder ungesättigten 5-, 6- oder 7-Ring bildet,
Q eine direkte Bindung oder eine Vinylen- oder eine Vinylidengruppe, die zusammen mit einem der beiden Reste R⁴ oder R⁵ einen Indolring oder bei Ausbildung einer exocyclischen Doppelbindung einen Indolinring bilden kann, und n 1 oder 2 ist,
und CH-aktiven Verbindungen mit den Formeln II und/oder III, 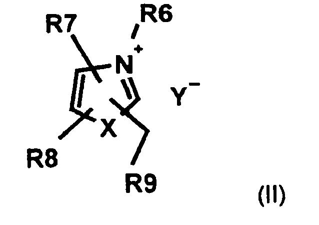
in der R⁶ eine C₁₋₁₀-Alkyl-, C₂₋₁₀-Alkenyl-, C₂₋₄-Hydroxyalkyl-, C₂₋₄-Carboxyalkyl-, C₂₋₄₋Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe >N-R¹², oder -CH=CH-, in denen R¹² für eine C₁₋₄-Alkyl-, C₂₋₄-Carboxalkyl-, C₂₋₄-Sulfoalkyl-, C₂₋₄-Sulfoxyalkyl-, C₂₋₄-Hydroxyalkyl- oder Aralkylgruppe, steht, ist und
Y- für ein Anion steht, das ausgewählt ist aus Halogenid, C₁₋₄-Alkylsulfat, C₁₋₄₋Alkansulfonat, Arensulfonat, C₁₋₄-Perfluoralkansulfat, Tetrafluorborat, Perhalogenat, Sulfat, Hydrogensulfat oder Carboxylat, 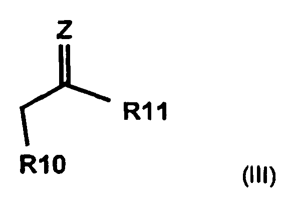
in der R¹⁰ für eine C₁₋₄-Acylgruppe, Aroyl-, C₁₋₄-Alkylsulfonyl-, C₁₋₄-Alkylsulfinyl-, C₁₋₄₋Alkylamino-, Di-C₁₋₄-Alkylamino-, Vinylcarbonyl-, Methinimino-, Nitril-, Ester oder Carbonsäureamidgruppe, die ggf. durch C₁₋₄-Alkyl-, C₂₋₄-Hydroxyalkyl oder Arylgruppen substituiert sein kann, steht und
R¹¹ für eine C₁₋₄-Acyl-, C₁₋₄-Alkoxy-, C₁₋₄-Alkylamino-, C₁₋₄-Acylamino- oder Di-C₁₋₄₋alkylaminogruppe steht,
wobei die Reste R¹⁰ und R¹¹ gemeinsam mit dem Restmolekül einen 5-, 6- oder 7-gliedrigen Cyclus aus der Reihe der 1,3-Thiazolidin-2,5-dione, 1,3-Thiazolidin-2-thion-5-one, Perhydropyrimidin-2,4,6-trione, Perhydropyrimidin-2-thion-4,6-dione, Cyclopentan-1,3-dione, Cyclohexan-1,3-dione, Indole, Indan-1,3-dione, Indan-1-one, 2-Pyrazolin-5-one, 1,2-Dihydro-6-hydroxy-2-hydroxypyridine oder deren Enolester bilden können,
und Z für Sauerstoff, Schwefel oder Dicyanmethylengruppe steht
und/oder Reaktionsprodukte aus diesen Verbindungen, ausgenommen Mittel, enthaltend 4-Dimethylaminobenzaldehyd als Verbindung gemäß Formel (I) in Kombination mit einer der CH-aktiven Verbindungen, ausgewählt aus der Gruppe, die gebildet wird aus 3-Ethyl-methylbenzothiazolium iodid, 3-Ethylmethylbenzoxazolium iodid, 1,2-Dimethylchinolinium iodid, 5-Chlor-3-ethyl-2-methylbenzothiazolium iodid, 2-Methyl-1-(3-sulfopropyl)-naphtho[1,2-d]thiazolium betain, 5-Methoxy-2-methyl-3-(3-sulfopropyl)benzothiazolium betain und 3-Amino-1-phenyl-pyrazolin-5-on.

2. Mittel nach Anspruch 1, **dadurch gekennzeichnet, daß** als Verbindungen mit der Formel I 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)benzaldehyd, 4-Diphenylamino-benzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-lmidazolyl)-benzaldehyd, 2-Morpholinobenzaldehyd, Indol-3-carboxaldehyd, 1-Methylindol-3-carboxaldehyd, N-Ethylcarbazol-3-aldehyd, sowie deren beliebigen Gemische eingesetzt werden.

3. Mittel nach Anspruch 1 oder 2, **dadurch gekennzeichnet, daß** als Verbindungen mit der Formel II 1,4-Dimethylchinolinium-, 1,2-Dimethylchinolinium-, 1,4-Dimethylpyridinium-, 1,2-Dimethylpyridinium-, 2-Methyl-1-ethylchinolinium-, 2,3-Di-methylisochinolinium-, 2,3-Dimethylbenzothiazolium-, 2,3-Dimethyl-6-nitrobenzothiazolium-, 3-Benzyl-2-benzothiazolium-, 2-Methyl-3-propylbenzothiazolium-, 2,4-Dimethyl-3-ethylthiazolium-, 3-(2-Carboxyethyl)-2,5-dimethylbenzothiazolium-, 1,2,3-Trimethylbenzimidazolium-, 5,6-Dichlor-1,3-diethyl-2-methylbenzimidazolium-, 3-Ethyl-2-methylbenzothiazolium-, 3-Ethyl-2-methylnaphtho[1,2-d]thiazolium-, 5-Chlor-3-ethyl-2-methylbenzothiazolium-, 3-Ethyl-2-methylbenzoxazolium-Salze, die z. B. als Chloride, Bromide, Iodide, Methansulfonate, Benzolsulfonate, p-Toluolsulfonate, Trifluormethansulfonate, Methylsulfate, Tetrafluorborate vorliegen können, sowie 2-Methyl-3-(3-sulfopropyl)-benzothiazoliumhydroxid, inneres Salz, 5-Methoxy-2-methyl-3-(3-sulfopropyl)-benzothiazoliurn-hydroxid, inneres Salz und beliebige Gemische der voranstehenden eingesetzt werden.

4. Mittel nach einem der Ansprüche 1 bis 3, **dadurch gekennzeichnet, daß** als CH-aktiven Verbindungen mit der Formel III Rhodanin, Rhodanin-3-essigsäure, Barbitursäure, Thiobarbitursäure, 1,3-Dimethyl-, 1,3-Diethylthiobarbitursäure, Oxindol, 3-Indoxylacetat, 1-Methyl-3-phenylpyrazolinon, Indan-1,3-dion, Cyclopentan-1,3-dion, 1,2-Dihydro-1-ethyl-6-hydroxy-4-methyl-2-oxo-3-pyridincarbonitril und beliebige Gemische der voranstehenden eingesetzt werden.

5. Mittel nach einem der Ansprüche 1 bis 4, **dadurch gekennzeichnet, daß** die Reaktionsprodukte aus den Verbindungen mit den Formeln I und II und/oder III ausgewählt sind aus Quinaldine red (2-[4-(Dimethylamino)-styryl]-1-ethylchinolinium-iodid), 2-[4-(Dimethylamino)-styryl]-1-methylchinolinium-iodid, 2-[4-(Dimethylamino)-styryl]-1-ethylpyridinium-iodid, 2-[4-(Dimethylamino)-styryl]-1-methylpyridinium-iodid, 4-[4-Dimethylaminostyryl]-1-methylpyridinium-tosylat, 4-[2-(2,3,6,7-Tetrahydro-1H,5H-benzo[ij]chinolizin-9-yl)-ethenyl]-1-methylpyridinium-iodid, trans-4-[4-(Dibutylamino)-styryl]-1-methylpyridiniumioidid und 2,6-Bis-[4-(dimethylamino)-styryl]-1-methylpyridinium-iodid, 2-[4-(Dimethylamino)-styryl]-3-methylbenzoxazolium-perchlorat, 2-[4-(Dimethylamino)-styryl]-3-methylbenzoxazolium-iodid, 2-[4-(Dimethylamino)-styryl-1-ethylnaphtho[1,2-d]thiazolium-iodid und beliebigen Gemischen der voranstehenden.

6. Mittel nach einem der Ansprüche 1 bis 5, **dadurch gekennzeichnet, daß** die aromatischen Aldehyde bzw. Ketone mit der Formel I und die quaternären heterocyclischen Verbindungen mit der Formel II und die CH-aktiven Verbindungen mit der Formel III jeweils in einer Menge von 0,03 bis 65 mmol, insbesondere von 1 bis 40 mmol, bezogen auf 100 g des gesamten Färbemittels, enthalten sind.

7. Mittel nach einem der Ansprüche 1 bis 6, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

8. Mittel nach einem der Ansprüche 1 bis 7, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

9. Mittel nach einem der Ansprüche 1 bis 8, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

10. Mittel nach einem der Ansprüche 1 bis 9, **dadurch gekennzeichnet, daß** es Oxidationsmittel, insbesondere H₂O₂, in einer Menge von 0,01 bis 6 Gew.-%, bezogen auf die Anwendungslösung, enthält.

11. Mittel nach einem der Ansprüche 1 bis 10, **dadurch gekennzeichnet, daß** es anionische, zwitterionische oder nichtionische Tenside enthält.

12. Verwendung eines Färbmittels gemäß Anspruche 1 als eine färbende Komponente in Oxidationshaarfärbemitteln.

13. Verfahren zum Färben von keratinhaltigen Fasern, insbesondere menschlichen Haaren, worin ein Färbemittel gemäß Anspruch 1 auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for dyeing keratin fibres, in particular human hair, comprising, as dyeing component, a combination of aromatic aldehydes or ketones with the formula I, 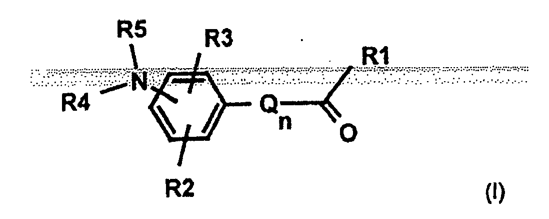
in which R¹ is a hydrogen atom, a C₁₋₄-alkyl group or an aryl group,
R², R³, independently of one another, are a hydrogen atom, a C₁₋₄-alkyl group, a halogen atom, a hydroxy group,
C₁₋₄-alkoxy group, nitro group or together are a fused-on aromatic ring,
R⁴ and R⁵ are a hydrogen atom, a C₁₋₄-alkyl group, C₂₋₄-hydroxyalkyl group, C₁-₄-alkenyl group or aryl group or, together with the nitrogen atom, form a five- to seven-membered heterocyclic ring, or one of them with one of the radicals R² or R³ forms a saturated or unsaturated 5-, 6- or 7-membered ring,
Q is a direct bond or a vinylene group or a vinylidene group which, together with one of the two radicals R⁴ or R⁵, can form an indole ring or, with formation of an exocyclic double bond, an indoline ring, and
n is 1 or 2,
and CH-active compounds with the formulae II and/or III, 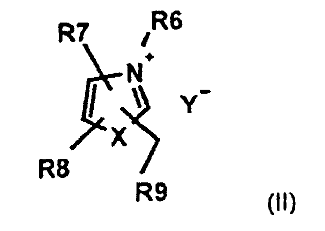
in which R⁶ a C₁₋₁₀-alkyl group, C₂₋₁₀-alkenyl group, C₂₋₄-hydroxyalkyl group,
C₂₋₄-carboxyalkyl group, C₂₋₄-sulphoalkyl group or aralkyl group,
R⁷ and R⁸, independently of one another, are a hydrogen atom, a C₁₋₄-alkyl group, a halogen atom, a hydroxy group, C₁₋₄-alkoxy group or nitro group or together form a fused-on aromatic ring,
R⁹ is a hydrogen atom, a C₁₋₄-alkyl group or an aryl group,
X is an oxygen or sulphur atom, the group >N-R¹², or -CH=CH-, in which R¹² is a C₁₋₄-alkyl, C₂₋₄-carboxyalkyl, C₂₋₄-sulphoalkyl, C₂₋₄-sulphoxyalkyl, C₂₋₄-hydroxyalkyl or aralkyl group, and
Y⁻ is an anion which is chosen from halide, C₁₋₄-alkyl sulphate, C₁₋₄-alkane-sulphonate, arenesulphonate, C₁₋₄-perfluoroalkane sulphate, tetrafluoroborate, perhalogenate, sulphate, hydrogen sulphate or carboxylate, 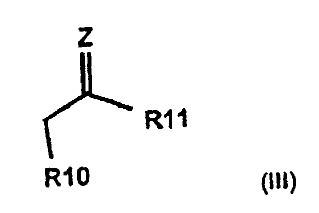
in which R¹⁰ is a C₁₋₄-acyl group, aroyl group, C₁₋₄-alkylsulphonyl group, C₁₋₄-alkylsulphinyl group, C₁₋₄-alkyl amino group, di-C₁₋₄-alkylamino group, vinylcarbonyl group, methinimino group, nitrile group, ester group or carboxamide group, which may be optionally substituted by C₁₋₄-alkyl, C₂₋₄-hydroxyalkyl or aryl groups, and
R¹¹ is a C₁₋₄-acyl group, C₁₋₄-alkoxy group, C₁₋₄-alkylamino group, C₁₋₄-acylamino group or di-C₁₋₄-alkylamino group,
where the radicals R¹⁰ and R¹¹, together with the remainder of the molecule, can form a 5-, 6- or 7-membered cycle from the series of 1,3-thiazolidine-2,5-diones, 1,3-thiazolidine-2-thion-5-ones, perhydropyrimidine-2,4,6-triones, perhydropyrimidine-2-thione-4,6-diones, cyclopentane-1,3-diones, cyclohexane-1,3-diones, indoles, indane-1,3-diones, indan-1-ones, 2-pyrazolin-5-ones, 1,2-dihydro-6-hydroxy-2-hydroxypyridines or enol esters thereof,
and Z is oxygen, sulphur or dicyanomethylene group
and/or reaction products of these compounds,
with the exception of agents comprising 4-dimethylaminobenzaldehyde as compound according to formula (I) in combination with one of the CH-active compounds chosen from the group which is formed from 3-ethylmethylbenzothiazolium iodide, 3-ethylmethylbenzoxazolium iodide, 1,2-dimethylquinolinium iodide, 5-chloro-3-ethyl-2-methylbenzothiazolium iodide, 2-methyl-1-(3-sulphopropyl)naphtho[1,2-d]thiazolium betaine, 5-methoxy-2-methyl-3-(3-sulphopropyl)benzothiazolium betaine and 3-amino-1-phenylpyrazolin-5-one.

2. Agent according to Claim 1, **characterized in that** the compounds with the formula I used are 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 4-piperidinobenzaldehyde, 4-dimethylaminoacetophenone, 4-dimethylaminonaphthaldehyde, 4-dimethylaminobenzylideneacetone, 4-dimethylaminocinnamaldehyde, 2-dimethylaminobenzaldehyde, 2-chloro-4-dimethylaminobenzaldehyde, 4-dimethylamino-2-methylbenzaldehyde, trans-4-diethylaminocinnamaldehyde, 4-(dibutylamino)benzaldehyde, 4-diphenylaminobenzaldehyde, 2,3,6,7-tetrahydro-1H,5H-benzo[ij]-quinoline-9-carboxaldehyde, 4-dimethylamino-2-methoxybenzaldehyde, 2,3,6,7-tetrahydro-8-hydroxy-1H,5H-benzo[ij]quinoline-9-carboxaldehyde, 4-(1-imidazolyl)benzaldehyde, 2-morpholinobenzaldehyde, indole-3-carboxaldehyde, 1-methylindole-3-carboxaldehyde, N-ethylcarbazole-3-aldehyde and any mixtures thereof.

3. Agent according to Claim 1 or 2, **characterized in that** the compounds with the formula II used are the salts of 1,4-dimethylquinolinium, 1,2-dimethylquinolinium, 1,4-dimethylpyridinium, 1,2-dimethylpyridinium, 2-methyl-1-ethylquinolinium, 2,3-dimethylisoquinolinium, 2,3-dimethylbenzothiazolium, 2,3-dimethyl-6-nitrobenzothiazolium, 3-benzyl-2-benzothiazolium, 2-methyl-3-propylbenzothiazolium, 2,4-dimethyl-3-ethylthiazolium, 3-(2-carboxyethyl)-2,5-dimethylbenzothiazolium, 2,2,3-trimethylbenzimidazolium, 5,6-dichloro-1,3-diethyl-2-methylbenzimidazolium, 3-ethyl-2-methylbenzothiazolium, 3-ethyl-2-methylnaphtho[1,2-d]thiazolium, 5-chloro-3-ethyl-2-methylbenzothiazolium, 3-ethyl-2-methylbenzoxazolium, which may be present, for example as chlorides, bromides, iodides, methanesulphonates, benzenesulphonates, p-toluenesulphonates, trifluoromethanesulphonates, methyl sulphates, tetrafluoroborates, and also 2-methyl-3-(3-sulphopropyl)benzothiazolium hydroxide, internal salt, 5-methoxy-2-methyl-3-(3-sulphopropyl)benzothiazolium hydroxide, internal salt and any mixtures of the above.

4. Agent according to one of Claims 1 to 3, **characterized in that** the CH-active compounds with the formula III used are rhodanine, rhodanine-3-acetic acid, barbituric acid, thiobarbituric acid, 1,3-dimethyl-, 1,3-diethylthiobarbituric acid, oxindole, 3-indoxyl acetate, 1-methyl-3-phenylpyrazolinone, indane-1,3-dione, cyclopentane-1,3-dione, 1,2-dihydro-1-ethyl-6-hydroxy-4-methyl-2-oxo-3-pyridinecarbonitrile and any mixtures of the above.

5. Agent according to one of Claims 1 to 4, **characterized in that** the reaction product from the compounds with the formulae I and II and/or III are chosen from quinaldine red (2-[4-(dimethylamino)styryl]-1-ethylquinolinium iodide), 2-[4-(dimethylamino)styryl]-1-methylquinolinium iodide, 2-[4-(dimethylamino)styryl]-1-ethylpyridinium iodide, 2-[4-(dimethylamino)styryl]-1-methylpyridinium iodide, 4-[4-dimethylaminostyryl]-1-methylpyridinium tosylate, 4- [2- (2, 3, 6, 7-tetrahydro-1H, 5H-benzo[ij]quinolizin-9-yl)ethenyl]-1-methylpyridinium iodide, trans-4-[4-(dibutylamino)styryl)-1-methylpyridinium iodide and 2,6-bis[4-(dimethylamine)styryl]-1-methylpyridinium iodide, 2-[4-(dimethylamino)styryl]-3-methylbenzoxazolium perchlorate, 2-[4-(dimethylamino)styryl]-3-methylbenzoxazolium iodide, 2-[4-(dimethylamino)styryl-1-ethylnaphtho[1,2-d]thiazolium iodide and any mixtures of the above.

6. Agent according to one of Claims 1 to 5, **characterized in that** the aromatic aldehydes or ketones with the formula I and the quaternary heterocyclic compounds with the formula II and the CH-active compounds with the formula III are in each case present in an amount of from 0.03 to 65 mmol, in particular from 1 to 40 mmol, based on 100 g of the total dyeing agent.

7. Agent according to one of Claims 1 to 6,
**characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

8. Agent according to one of Claims 1 to 7, **characterized in that** it comprises direct dyes from the group of nitrophenylenediamines, nitroaminophenols, anthraquinones or indolephenols, preferably in an amount of from 0.01 to 20% by weight, based on the total dyeing agent.

9. Agent according to one of Claims 1 to 8, **characterized in that** ammonium salts or metal salts chosen from the group of formates, carbonates, halides, sulphates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

10. Agent according to one of Claims 1 to 9, **characterized in that** it comprises oxidizing agents, in particular H₂O₂, in an amount of from 0.01 to 6% by weight, based on the application solution.

11. Agent according to one of Claims 1 to 10, **characterized in that** it comprises anionic, zwitterionic or nonionic surfactants.

12. Use of a dyeing agent according to Claim 1 as a dyeing component in oxidation hair colorants.

13. Method of dyeing keratin fibres, in particular human hair, in which a dyeing agent according to Claim 1 is applied to the keratin fibres, left for a time, usually about 30 minutes, on the fibres and then rinsed out again or washed out with a shampoo.

## Revendications

1. Agent pour la teinture de fibres kératiniques, en particulier de cheveux humains, contenant, à titre de composant de coloration, une combinaison d'aldéhydes, respectivement de cétones aromatiques répondant à la formule I, 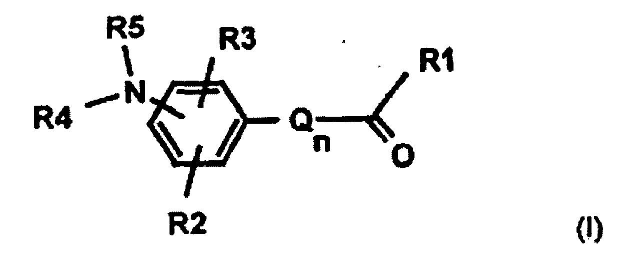
où R¹ représente un atome d'hydrogène, un groupe alkyle en C₁-C₄ ou un groupe aryle ;
R², R³ représentent, indépendamment l'un de l'autre, un atome d'hydrogène, un groupe alkyle en C₁-C₄, un atome d'halogène, un groupe hydroxyle, un groupe alcoxy en C₁-C₄, un groupe nitro, ou représentent ensemble un noyau aromatique condensé,
R⁴ et R⁵ représentent un atome d'hydrogène, un groupe alkyle en C₁-C₄, un groupe hydroxyalkyle en C₂-C₄, un groupe alcényle en C₁-C₄ ou un groupe aryle, ou forment, ensemble avec l'atome d'azote, un noyau hétérocyclique contenant de cinq à sept membres, ou bien un des deux radicaux forme, avec un desdits radicaux R² ou R³, un noyau saturé ou insaturé contenant 5, 6 ou 7 membres,
Q représente une liaison directe ou un groupe vinylène ou un groupe vinylidène qui, ensemble avec un des deux radicaux R⁴ ou
R⁵ peut former un noyau indole ou, lors de la réalisation d'une liaison double exocyclique, un noyau indoline, et n est égal à 1 ou 2,
et de composés contenant des groupes CH actifs, répondant aux formules II et/ou III, 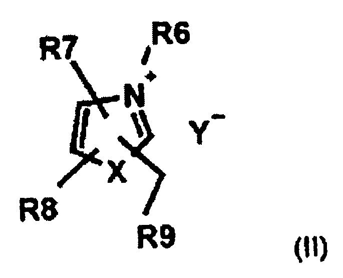
où R⁶ représente un groupe alkyle en C₁-C₁₀, un groupe alcényle en C₂-C₁₀, un groupe hydroxyalkyle en C₂-C₄, un groupe carboxyalkyle en C₂-C₄, un groupe sulfoalkyle en C₂-C₄ ou un groupe aralkyle,
R⁷ et R⁸ représentent, indépendamment l'un de l'autre, un atome d'hydrogène, un groupe alkyle en C₁-C₄, un atome d'halogène, un groupe hydroxyle, un groupe alcoxy en C₁-C₄ ou un groupe nitro, ou forment ensemble un noyau aromatique condensé,
R⁹ représente un atome d'hydrogène, un groupe alkyle en C₁-C₄ ou un groupe aryle,
X représente un atome d'oxygène ou un atome de soufre, le groupe >N-R¹² ou un groupe -CH=CH-, R¹² représentant un groupe alkyle en C₁-C₄, un groupe carboxyalkyle en C₂-C₄, un groupe sulfoalkyle en C₂-C₄, un groupe sulfoxyalkyle en C₂-C₄, un groupe hydroxyalkyle en C₂-C₄ ou un groupe aralkyle, et
Y⁻ représente un anion qui est choisi parmi le groupe comprenant un halogénure, un alkyl(en C₁-C₄)sullfate, un alcane(en C₁₋C₄)sulfonate, un arènesulfonate, un perfluoroalcane(en C₁-C₄)-sulfate, un tétrafluoroborate, un perhalogénate, un sulfate, un hydrogénosulfate ou un carboxylate 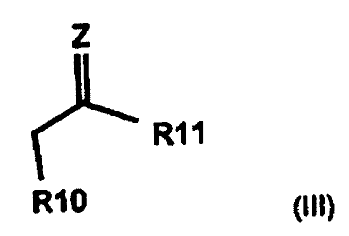
où R¹⁰ représente un groupe acyle en C₁-C₄, un groupe aroyle, un groupe alkyl(en C₁-C₄)sulfonyle, un groupe alkyl(en C₁-C₄)-sulfinyle, un groupe alkyl(en C₁-C₄)amino, un groupe dialkyl(en C₁-C₄)amino, un groupe vinylcarbonyle, un groupe méthine-imino, un groupe nitrile, un groupe ester ou un groupe amide d'acide carboxylique qui peut éventuellement être substitué par un ou plusieurs groupes alkyle en C₁-C₄, hydroxyalkyle en C₂-C₄ ou aryle, et
R¹¹ représente un groupe acyle en C₁-C₄, un groupe alcoxy en C₁₋C₄, un groupe alkyl(en C₁-C₄)amino, un groupe acyl(en C₁₋C₄)amino ou un groupe dialkyl(en C₁-C₄)amino,
les radicaux R¹⁰ et R¹¹ pouvant former, ensemble avec la molécule résiduelle, un cycle contenant 5, 6 ou 7 membres choisi parmi la série des 1,3-thiazolidine-2,5-diones, des 1,3-thiazolidine-2-thion-5-ones, des perhydropyrimidine-2,4,6-triones, des perhydropyrimidine-2-thione-4,6-diones, des cyclopentane-1,3-diones, des cyclohexane-1,3-diones, des indoles, des indane-1,3-diones, des indan-1-ones, des 2-pyrazolin-5-ones, des 1,2-dihydro-6-hydroxy-2-hydroxypyridines ou de leurs esters énoliques,
et Z représente un atome d'oxygène, un atome de soufre ou un groupe dicyanométhylène
et/ou des produits réactionnels de ces composés, à l'exception d'agents contenant du 4-diméthylaminobenzaldéhyde à titre de composé répondant à la formule (I) en combinaison avec un des composés contenant des groupes CH actifs, choisis parmi le groupe qui est formé par l'iodure de 3-éthyl-méthylbenzothiazolium, l'iodure de 3-éthylméthylbenzoxazolium, l'iodure de 1,2-diméthylquinolinium, l'iodure de 5-chloro-3-éthyl-2-méthylbenzothiazolium, la bétaïne de 2-méthyl-1-(3-sulfopropyl)-naphto[1,2-d]thiazolium, la bétaïne de 5-méthoxy-2-méthyl-3-(3-sulfopropyl)benzothiazolium et la 3-amino-1-phényl-pyrazolin-5-one.

2. Agent selon la revendication 1, **caractérisé en ce qu'**on met en oeuvre, à titre de composés répondant à la formule I, le 4-diméthylaminobenzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, le 4-pipéridinobenzaldéhyde, la 4-diméthylaminoacétophénone, le 4-diméthylaminonaphtaldéhyde, la 4-diméthylaminobenzylidène-acétone, le 4-diméthylaminocinnamaldéhyde, le 2-diméthylaminobenzaldéhyde, le 2-chloro-4-diméthylaminobenzaldéhyde, le 4-diméthylamino-2-méthylbenzaldéhyde, le trans-4-diéthylaminocinnamaldéhyde, le 4-(dibutylamino)benzaldéhyde, le 4-diphénylaminobenzaldéhyde, le 2,3,6,7-tétrahydro-1H,5H-benzo[i,j]quinolizine-9-carboxaldéhyde, le 4-diméthylamino-2-méthoxybenzaldéhyde, le 2,3,6,7-tétrahydro-8-hydroxy-1H,5H-benzo[i,j]quinolizine-9-carboxaldéhyde, le 4-(1-imidazolyl)-benzaldéhyde, le 2-morpholinobenzaldéhyde, l'indole-3-carboxaldéhyde, le 1-méthylindol-3-carboxaldéhyde, le N-éthylcarbazol-3-aldéhyde, ainsi que l'un quelconque de leurs mélanges.

3. Agent selon la revendication 1 ou 2, **caractérisé en ce qu'**on met en oeuvre, à titre de composés répondant à la formule II, des sels du 1,4-diméthylquinolinium, du 1,2-diméthylquinolinium, du 1,4-diméthylpyridinium, du 1,2-diméthylpyridinium, du 2-méthyl-1-éthylquinolinium, du 2,3-diméthylisoquinolinium, du 2,3-diméthylbenzothiazolium, du 2,3-diméthyl-6-nitrobenzothiazolium, du 3-benzyl-2-benzothiazolium, du 2-méthyl-3-propylbenzothiazolium, du 2,4-diméthyl-3-éthylthiazolium, du 3-(2-carboxyéthyl)-2,5-diméthylbenzothiazolium, du 1,2,3-triméthylbenzimidazolium, du 5,6-dichloro-1,3-diéthyl-2-méthylbenzimidazolium, du 3-éthyl-2-méthylbenzothiazolium, du 3-éthyl-2-méthylnaphto[1,2-d]-thiazolium, du 5-chloro-3-éthyl-2-méthylbenzothiazolium, du 3-éthyl-2-méthylbenzoxazolium, qui peuvent se présenter sous la forme de chlorures, de bromures, d'iodures, de méthanesulfonates, de benzènesulfonates, de p-toluènesulfonates, de trifluorométhanesulfonates, de méthylsulfates, de tétrafluoroborates, ainsi que l'hydroxyde du 2-méthyl-3-(3-sulfopropyl)-benzothiazolium, sel interne, l'hydroxyde du 5-méthoxy-2-méthyl-3-(3-sulfopropyl)-benzothiazolium, sel interne et l'un quelconque des mélanges des sels ci-dessus.

4. Agent selon l'une quelconque des revendications 1 à 3, **caractérisé en ce qu'**on met en oeuvre à titre de composés contenant des groupes CH actifs répondant à la formule III, la rhodanine, l'acide rhodanine-3-acétique, l'acide barbiturique, l'acide thiobarbiturique, l'acide 1,3-diméthylthiobarbiturique, l'acide 1,3-diéthylthiobarbiturique, l'oxindole, l'acétate de 3-indoxyle, la 1-méthyl-3-phénylpyrazolinone, l'indane-1,3-dione, la cyclopentane-1,3-dione, le 1,2-dihydro-1-éthyl-6-hydroxy-4-méthyl-2-oxo-3-pyridinecarbonitrile et l'un quelconque des mélanges des composés ci-dessus.

5. Agent selon l'une quelconque des revendications 1 à 4, **caractérisé en ce que** les produits réactionnels des composés répondant aux formules I et II et/ou III sont choisis parmi le groupe comprenant le rouge de quinaldine (iodure du 2-[4-(diméthylamino)-styryl]-1-éthylquinolinium), l'iodure du 2-(4-(diméthylamino)-styryl]-1-méthylquinolinium, l'iodure du 2-[4-(diméthylamino)-styryl]-1-éthylpyridinium, l'iodure du 2-[4-(diméthylamino)-styryl]-1-méthylpyridinium, le tosylate du 4-[4-diméthylaminostyryl]-1-méthylpyridinium, l'iodure du 4-[2-(2,3,6,7-tétrahydro-1H,5H-benzo[i,j]quinolizine-9-yl)-éthényl]-1-méthylpyridinium, l'iodure du trans-4-[4-(dibutylamino)-styryl]-1-méthylpyridinium et l'iodure du 2,6-bis-[4-(diméthylamino)-styryl]-1-méthylpyridinium, le perchlorate du 2-[4-(diméthylamino)-styryl]-3-méthylbenzoxazolium, l'iodure du 2-[4-(diméthylamino)-styryl]-3-méthylbenzoxazolium, l'iodure du 2-[4-(diméthylamino)-styryl-1-éthylnaphto[1,2-d]thiazolium et l'un quelconque des mélanges des composés ci-dessus.

6. Agent selon l'une quelconque des revendications 1 à 5, **caractérisé en ce que** les aldéhydes, respectivement les cétones aromatiques répondant à la formule I et les composés hétérocycliques quaternaires répondant à la formule II et les composés contenant des groupes CH actifs répondant à la formule III sont contenus respectivement dans des quantités de 0,03 à 65 millimoles, en particulier de 1 à 40 millimoles, rapportés à 100 g de la teinture totale.

7. Agent selon l'une quelconque des revendications 1 à 6, **caractérisé en ce qu'**il contient un agent de renforcement des couleurs choisi parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

8. Agent selon l'une quelconque des revendications 1 à 7, **caractérisé en ce qu'**il contient des colorants montant directement sur la fibre, choisis parmi le groupe comprenant des nitrophénylènediamines, des nitroamino-phénols, des anthraquinones ou des indophénols, de préférence en une quantité de 0,01 à 20 % en poids, rapportés à la teinture totale.

9. Agent selon l'une quelconque des revendications 1 à 8, **caractérisé en ce qu'**on ajoute des sels d'ammonium ou des sels métalliques choisis parmi le groupe comprenant les formiates, les carbonates, les halogénures, les sulfates, les butyrates, les valérates, les caproates, les acétates, les lactates, les glycolates, les tartrates, les citrates, les gluconates, les propionates, les phosphates et les phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

10. Agent selon l'une quelconque des revendications 1 à 9, **caractérisé en ce qu'**il contient, des agents d'oxydation, en particulier du peroxyde d'hydrogène, en une quantité de 0,01 à 6 % en poids, rapportés à la solution d'utilisation.

11. Agent selon l'une quelconque des revendications 1 à 10, **caractérisé en ce qu'**il contient des agents tensioactifs anioniques, zwitterioniques ou non ioniques.

12. Utilisation d'une teinture selon la revendication 1, à titre de composant de coloration dans des teintures capillaires par oxydation.

13. Procédé pour la teinture de fibres kératiniques, en particulier de cheveux humains, dans lequel on applique, sur les fibres kératiniques, une teinture selon la revendication 1, on l'abandonne pendant un certain temps, habituellement pendant environ 30 minutes sur les fibres, avant de l'éliminer par rinçage ou par lavage avec un shampooing.
